# EUROPEAN PATENT APPLICATION

(11) **EP 1 108 429 A2**
(43) Date of publication of application: **20.06.2001**
(21) Application number: 00311079.8
(22) Date of filing: 13.12.2000
(51) Int. Cl.: A61K 31/198, A61K 9/00, A61K 9/06, A61K 47/36, A61P 3/00

(54) **An improved amino acid composition for providing an amino acid supplement or protein substitute, particularly for the treatment and/or management of certain diseases**

(30) Priority: 14.12.1999 GB 9929497
(71) Applicant: Vitaflo Limited, Liverpool L3 4BL (GB)
(72) Inventor: O'Donnell, Maura, Hightown, Merseyside L38 3RT (GB); Partington, Thomas, Liverpool, Merseyside L3 4EE (GB)
(74) Representative: Lees, Kate Jane

(57) **Abstract**

An amino acid composition for administration to a patient for the treatment and/or management of a disease comprises an amino acid or a specific combination of amino acids suitable for treatment of a particular disease and a thickening agent, such as a pre-gelatinised starch, modified starch, gum and/or cellulose. Addition of an aqueous liquid to the mixture provides a smooth gel-like substance for consumption by the patient. For example, the amino acid mixture may contain an amino acid profile corresponding to normal dietary protein but excluding phenylalanine for administering to sufferers of phenylketonuria.

## Description

The present invention relates to an improved amino acid composition for providing an amino acid supplement or protein substitute for the treatment and/or management of certain diseases, particularly but not exclusively for the treatment and/or management of diseases of amino acid metabolism, such as phenylketonuria.

Phenylketonuria (PKU) is a group of recessively inherited disorders characterised by a deficiency of the liver enzyme phenylalanine hydroxylase which is necessary for the breakdown of the essential amino acid phenylalanine to tyrosine. As a result of this deficiency, plasma phenylalanine concentrations rise and abnormal metabolites are excreted in the urine.

The incidence of PKU in the U.K. is 1 in 10,000 births, in Ireland it is 1 in 4,500. Since 1968 all infants in the U.K. have been screened for PKU between the 6^{th} and 14^{th} day after birth by measuring the level of phenylalanine in the plasma.

Children that suffer from PKU produce high levels of toxins from the intake of a normal protein diet which leads to mental retardation, seizures and defects in pigmentation. PKU is treated by the administration of a low phenylalanine diet, which should be introduced as soon as possible after diagnosis. Phenylalanine intake is reduced so that plasma phenylalanine is in turn reduced to the plasma reference range 100-120 µmol/l. The evidence so far suggests that PKU children treated in this way can grow up normally without any of the symptoms of PKU.

Phenylalanine is present in all dietary proteins and therefore, in order to lower the intake of this amino acid in PKU patients, it is necessary to substitute their dietary protein with a mixture of synthetic amino acids which has a similar amino acid profile to dietary protein but lacks phenylalanine. The resultant mixture of amino acids works very well in nutritional terms but has one major drawback in that it is a white powder with an extremely bad taste. This tends to lead to poor compliance with the dietary regime which in turn leads to less effective control of plasma phenylalanine levels and possible brain damage.

Manufacturers of such dietary products have therefore endeavoured to improve the taste and presentation of the amino acids mixture but the problem still remains a serious one. The current preferred method of alleviating the problem is to present the amino acid mixture as a citrus drink wherein the patient adds water to the mixture to create, for example, an orange flavoured drink.

However, this method is still not ideal. A large number of patients still dislike the taste and therefore do not want to drink such a large volume of the mixture, particularly with the younger patient. MacDonald et al (1997; Journal of Nutrition and Dietetics 10:163-70) reported that in young children it can take on average one hour a day to drink the protein substitute. As a result, some parents of children with PKU have made up the orange drink into a crude paste instead of the liquid drink, as directed. This overcomes the problem of the large volume of the drink and enables the parent to ensure that the correct dose is taken by the child but the paste still has a number of severe drawbacks making its consumption unpleasant for the patient.

The product was designed to be used at a much greater volume as a drink and therefore the flavour of the paste made up from the mixture is overly intense. The paste is also difficult to wet and has a gritty texture. Furthermore, the paste sticks to the walls of the mixing vessel which makes it difficult to ensure that the patient receives the required dosage of the mixture.

Existing products also require careful weighing, measuring and mixing which makes transport and administration difficult, especially for patients at school or in a social environment.

It is an object of the present invention to provide an improved amino acid composition for providing an amino acid supplement or protein substitute for the treatment and/or management of diseases, particularly diseases of amino acid metabolism, that aim to overcome the abovementioned drawbacks.

Accordingly, the present invention provides an amino acid composition for administration to a patient for the treatment and/or management of a disease, the composition comprising an amino acid or a specific combination of amino acids suitable for treatment of the particular disease and a thickening agent, such that addition of an aqueous liquid to the composition provides a smooth gel-like substance for consumption by the patient.

It is to be appreciated that the specific combination of amino acids will depend upon the disease to be treated. For example, in the case of Phenylketonuria, a synthetic amino acid mixture is provided that has a similar amino acid profile to normal dietary protein but does not include phenylalanine. Alternatively, the substitute may comprise a single amino acid, such as cystine or tyrosine with a thickening agent to provide an amino acid supplement.

The thickening agent may be one or a combination of a number of substances. Preferably, the agent is included in an amount of 1% to 40%, depending upon the particular type of agent used.

The thickening agent may be pre-gelatinised starch. Preferably, the pre-gelatinised starch is included in an amount of at least 5%, preferably not more than 35%.

Alternatively, the thickening agent may be a modified starch, preferably included in an amount of at least 5%, preferably not more than 35%. For example, the modified starch may be a starch ester, such as an acylated distarch adipate (E1422) or a phosphate, such as hydroxypropyle di-starch phosphate (E1442), an oxidised starch (E1404), a mono starch phosphate (E1410), a di-starch phosphate (E1412), a phosphated di-starch phosphate (E1413), an acetylated di-starch phosphate (E1414), an acetylated starch (E1420) or starch sodium octenyl succinate (E1450).

The thickening agent may alternatively be comprised of one or a combination of gums, such as locust bean gum (E410), guar gum (E412), Acacia gum (E414), Xanthan gum (E415) or pectins (E440). The gums are preferably provided in an amount not less than 1%, preferably not more than 5%.

Alternatively, the thickening agent may be made up of one or a combination of any celluloses in an amount not less than 1%, but not more than 5%. For example, celluloses (E460), methyl cellulose (E461) or others, such as E463, E464, E465 or E466.

The amino acid compositions of the present invention may be used as an amino acid supplement or as an protein substitute. The protein substitutes according to the present invention may be used to treat and manage any diseases that are conventionally managed using a substitute amino acid mixture in place of an ordinary protein diet. Such diseases include Maple Syrup Urine Disease, Tyrosinaemia, Methylmalonic acidaemia, Homocystinurea, Glutaric aciduria, Isovaleric acidaemia, Hyperlysinaemia, Alcaptonuria, Urea cycle Disorders, Propionic acidemia, Cows milk protein allergy, short gut syndrome and gut resection patients.

The composition may also include other standard nutrients, such as minerals, trace elements, vitamins and flavourings (such as raspberry or orange flavours) in amounts dependent upon the recommended safety levels and adequate daily dietary intakes.

Generally, the liquid that will be added to the composition is water. The gel-like substance may be used as a stand alone product or may be added to permitted foods. The composition may be provided in sachets, for example 20g sachets containing 8.4g protein equivalent or lOg amino acids. Preferably, 25-35 ml water is added to the powder, more preferably 30ml but more could be added to form a drink. The substance may be used as a spread, such as a savoury or sweet spread, which can be taken with a low protein bread or crackers or may be flavoured and made up as a dessert, such as crème brulee, crème caramel, custard and so on. The substance is particularly useful for "weaning" situations due to the texture being suitable for the 6 month to 12 month child.

The composition preferably has a protein carbohydrate ratio of approximately 1:1.

The present invention will now be illustrated with reference to the following Examples in which Example 1 details the constituents of an amino acid mixture for providing a protein substitute according to one embodiment of the present invention and investigates its administration to PKU patients; Example 2 compares the use of the protein substitute of Example 1 with the use of two earlier products used for the management of PKU; Example 3 investigates the shelf life of the protein substitute of Example 1 and Example 4 further investigates the efficacy and acceptability of the protein substitute of Example 1 in patients suffering from PKU; and with reference to the accompanying drawings in which:-
Figure 1 is a graph illustrating the daily weight of protein substitute/vitamin and mineral powders taken by the subjects before and after being administered the mixture of the present invention;
Figure 2 is a table listing all the nutrients taken by each of the subjects over the study period;
Figure 3 is a graph illustrating the nutrient intake of each of the subjects over the study period;

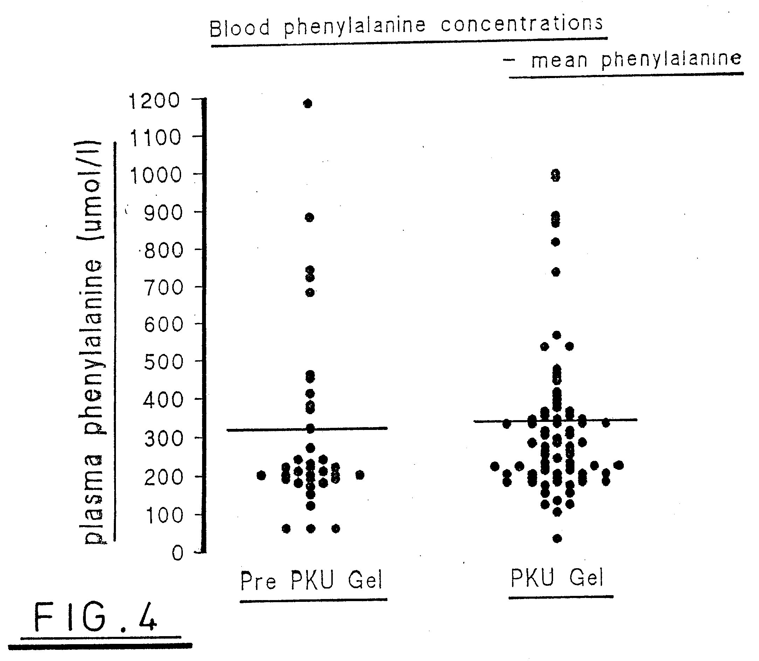

Figure 4 is a graph illustrating the plasma phenylalanine concentrate of the subjects four weeks prior to the study and during the eight weeks of the study;
Figure 5 is a table of the blood results of the subjects treated with the protein substitute of the present invention at week 0 and week 8; and
Figure 6 is a graph illustrating the weight and height Z scores of the subjects at the beginning and end of the study.

### Example 1

A new protein substitute, known as PKU gel^{TM}, having an amino acid profile similar to that of normal dietary protein but lacking phenylalanine and including a thickening agent, consisting of a mixture of pre-gelatinised starch and an acylated distarch adipate (E1422), together with other standard nutrients, such as minerals, trace elements and vitamins, was investigated to establish whether the composition was acceptable for administration to PKU patients.

Tables 1 to 5 below provide details of the energy and nutrient content of the composition.

**Table 1**

| | | **Per 100g** |
|---|---|---|
| Energy | **Kcal** **kj** | **340.05** **1421.96** |
| Protein equivalent | **g** | 42.0 |
| Carbohydrate | **g** | 43.02 |
| Fat | **g** | 0.00 |
| Moisture | **g** | 0.00 |

The nitrogen source in PKU-gel consists of pharmaceutical grade cystalline L-aminoacids.

Carbohydrate contributes approximately 50.3% of the energy, and the source of carbohydrate is sucrose and maize starch. Fat contributes less than 0.05% of the energy.

**Table 2**

| **Minerals** | | |
|---|---|---|
| Sodium | Mg | 378.88 |
| | *mmol* | *16.29* |
| Potassium | mg | 932.23 |
| | *mmol* | *24. 24* |
| Chloride | mg | 582.31 |
| | *mmol* | *16.30* |
| Calcium | mg | 542.64 |
| | *mmol* | *13.57* |
| Phosphorous | mg | 495.32 |
| | *mmol* | *15.85* |
| Magnesium | mg | 167.40 |
| | *mmol* | *6.86* |

**Table 3**

| **Trace Elements** | | |
|---|---|---|
| Iron | Mg | 10.50 |
| Zinc | Mg | 8.00 |
| Iodine | Mcg | 120.00 |
| Manganese | Mg | 1.75 |
| Copper | Mg | 0.70 |
| Molybdenum | Mcg | 50.00 |
| Selenium | Mcg | 30.00 |
| Chromium | Mcg | 70.00 |

**Table 4**

| **Vitamins** | | |
|---|---|---|
| Vitamin A | mcg RE | 600.00 |
| Vitamin E | mg alpha TE | 9.00 |
| Vitamin C | mg | 45.00 |
| Thiamin | mg | 0.80 |
| Riboflavin | mg | 1.00 |
| Vitamin B6 | mg | 1.10 |
| Niacin | mg NE | 13.50 |
| Pantothenic acid | mg | 3.75 |
| Myo-inositol | mg | 0.00 |
| Choline hydrochloride | mg | 0.00 |
| Vitamin D | mcg | 12.00 |
| Cyanocobalamin | mcg | 1.00 |
| Folic Acid | mcg | 130.00 |
| d-Biotin | mcg | 25.00 |
| Vitamin K1 | mcg | 25.00 |
| Beta Carotine | mg | 0.00 |
| Vitamin B12 | mcg | 1.00 |

Table 5 below shows the amino acid profile of the composition.

**Table 5**

| **Amino Acids** | | **Per 100g** |
|---|---|---|
| L-Alanine | g | 1.80 |
| L-Arginine | g | 1.91 |
| L-Aspartic acid | g | 4.44 |
| L-Cystine | g | 1.12 |
| L-Glutamic acid | g | 0.00 |
| Glycine | g | 5.38 |
| L-Histidine | g | 1.80 |
| L-iso Leucine | g | 3.14 |
| L-Leucine | g | 4.94 |
| L-Lysine | g | 3.28 |
| L-Methionine | g | 0.76 |
| L-Proline | g | 4.48 |
| L-Phenylalanine | g | 0.00 |
| L-Serine | g | 2.06 |
| L-Threonine | g | 3.14 |
| L-Tryptophan | g | 0.99 |
| L-Tyrosine | g | 4.04 |
| L-Valine | g | 3.59 |
| L-Asparagine | g | 0.00 |
| L-Citruline | g | 0.00 |
| L-Carnitine | mg | 45.14 |
| Taurine | mg | 90.07 |
| L-Glutamine | mg | 3587.54 |

The specific ingredients of the protein substitute are as follows:-
Sugar, Starch, L-Lysine L-aspartate, Glycine, Modified starch, L-Leucine, L-Tyrosine, L-Proline, L-Valine, L-Glutamine, L-Arginine L-Aspartate, L-Threonine, L-Isoleucine, L-Serine, L-Histidine, L-Alanine, Calcium phosphate, Potassium citrate, Magnesium phosphate, L-Cystine, Sodium citrate, L-Methionine, Potassium chloride, L-Trytophan, Sodium chloride, Ferrous gluconate, L-Taurine, Zinc gluconate, Ascorbic acid, L-Carnitine, Manganese gluconate, Nicotinamide, Copper gluconate, Pantothenic acid, Pyridoxine hydrochloride, Riboflavin, Thiamin hydrochloride, Vitamin A acetate, Potassium iodide, Folic acid, Chromium polynicolate, Ammonium molybdate, Seleno methionine, Vitamin Kl, D-Biotin, Vitamin D3, D alpha Tocopherol acetate, Cyanocobalamin.

The composition includes a thickening agent comprised of a mixture of pregelanised starch and an acylated distarch adipate (E1422). Such thickening agents have not previously been used in relation to the production of amino acid mixtures for the treatment and management of diseases such as PKU but have been used with drinks and foods that are administered to patients suffering from Dysphagia, a term used to describe difficulties with the swallowing reflex.

The use of the thickening agent with the amino acid mixture was surprisingly found to produce a gel that is easily wetted and has a smooth texture. Unexpectedly, the mouth-feel and taste of the product was also improved. Additionally, the nature of the gel was also thick yet free flowing and therefore the mixture is much easier to handle and does not stick to the sides of a mixing vessel. Accordingly, the present invention provides an amino acid mixture that may be administered to a patient in smaller volumes than the prior art citrus drinks but that does not have any of drawbacks associated with the crude paste. The substitute is particularly useful in weaning situations since the texture of the gel is suitable for a 6 month to 12 month child.

A patient evaluation study was carried out at Birmingham Children's Hospital where one of the UK's largest PKU clinics is held. The children and parents attending this clinic were given the new phenylalanine free protein substitute of the present invention and asked to compare its taste, texture, appearance, smell and volume with their present formulation.

10 patients were tested. The results are given in Table 6 below:-

**Table 6**

| | **Excellent** | **V. Good** | **Same** | **Poor** | **V. Poor** |
|---|---|---|---|---|---|
| **Taste** | 41.6% | 33.3% | 16.6% | - | 8.3% |
| **Texture** | 75% | 16.6% | - | - | 8.3% |
| **Appearance** | 90.0% | - | - | - | 9.1% |
| **Smell** | 81.8% | 18.2% | - | - | - |
| **Volume** | 66.6% | - | 25% | - | 8.3% |

### Example 2

The use of the protein substitute PKU-gel^{TM} of Example 1 in PKU patients was compared with two earlier products on the market, namely XP-Maxamaid^{TM} and Phlexy-10^{TM}. XP-Maxamaid^{TM}, unlike PKU-gel^{TM}, is not suitable for patients under one year of age and is 40% less concentrated in amino acids. Phlexy-10^{TM} is also not suitable for patients under one year of age. However, whilst it is as concentrated as PKU-gel^{TM}, it is nutritionally incomplete with children having to take separate vitamin and mineral supplements that are often difficult to consume.

Table 7 below compares the content of the three products and Table 8 below compares the weight and volumes of PKU-gel^{TM} and XP-Maxamaid^{TM} that have to be consumed by children to obtain the required amount.

**Table 7**

| | **PKU GEL**^{**TM**} | **PHLEXY-10**^{**TM**} | **XP-MAXAMAID**^{**TM**} |
|---|---|---|---|
| Rec, age group | 6mths-10yrs | 1 year + | 1 - 8yrs. |
| Amino acid conc. | 50% | 50% | 50% |
| Nutritionally complete | YES | NO | YES |
| Flavours | Unflavoured/raspberry/orange | Blackcurrant/apple | Unflavoured/orange |

**Table 8**

| **PKU-gel**^{**TM**} **G** | **XP-Maxamaid**^{**TM**} **G** | **PKU-gel**^{**TM**} **ml*** | **XP-Maxamaid**^{**TM**} **ml*** |
|---|---|---|---|
| 65 | 110 | 146 | 550 |
| 80 | 130 | 180 | 650 |
| 90 | 150 | 203 | 750 |
| 100 | 160 | 225 | 800 |

| | | | |
|---|---|---|---|
| * made up as per manufacturers' instructions. | | | |

As is evident from Table 8 above, the patients have to consume a far lower volume of product with the composition of the present invention compared to the prior art product XP-Maxamaid^{TM}.

### Example 3

The shelf life of PKU-gel^{TM} was investigated. Samples of manufactured packs were stored at ambient temperature (min recorded temperature = 19°C, max = 24°C) and were tested for microbiology, moisture, and Vitamin E soon after packaging and at 3 month intervals. Sensory evaluation was carried out initially after 1 month, 2 months and then every 3 months. There was no significant change in any parameter after 12 months; hence a 12 month shelf life is recommended for the product.

### Example 4

A further study was conducted to assess the acceptability and effectiveness of PKU-gel^{TM} in a group of patients with PKU aged between 6 months and 10 years. Nine children with PKU were recruited into the study from Birmingham Children's Hospital. There were 6 female (67%) and 3 males (33%) with a median age of 4 years (range from 1 to 10 years). The subjects had each been under active dietary follow-up for at least 3 months before entering the trial, had no known allergy to corn starch and a carer with the ability to take skin puncture blood specimens.

All the subjects were on a low phenylalanine diet comprising: 1) a dietary phenylalanine allocation using a 50 mg phenylalanine exchange system (50 mg ≈ 1g protein); 2) a phenylalanine - free protein substitute; and 3) low phenylalanine foods e.g. most fruits and vegetables permitted in normal quantities. All the subjects were aiming to keep their plasma phenylalanine concentrations within the MRC Working Group for PKU guidelines (MRC 1993) i.e. subjects 0-4 years: 100 µmol/l- 350 µmol/l; and subjects 5-10 years: 100 µmol/l-450 µmol/l. The median number of 50-mg phenylalanine exchanges allocated was five per day (range 4 to 10), or 5 g natural protein. The following amounts of total protein from protein substitute and phenylalanine exchanges were allocated: age <2 years: 3.0g/kg/day; and 2-5 years: 2.5g/kg/day. Their usual protein substitutes before entering the trial were: XP-Maxamaid^{TM} (n=4), XP-Maxamaid^{TM} and Aminogran Food Supplement (n=2) and Phlexy-10 drink mix (n=3). The three subjects on Phlexy-10^{TM} drink mix also took Paediatric Seravit as a comprehensive vitamin and mineral mixture.

The trial was conducted at home. Subjects were transferred to the new PKU gel^{TM} for an 8-week period to provide their main source of protein substitute. They could take the gel either as a paste or drink. The gel was given in 3 equal doses throughout the day. The patients were assessed as follows.

### (a) Assessment of dietary intake

Carers weighed and recorded their child's food intake over 3 days at the trial entry and during the week 8. Parents recorded only food actually eaten, and any waste food was reweighed and deducted from the total weight. They also described cooking techniques, precise food brand, kept nutritional labels of manufactured foods eaten, and identified individual ingredients of any home made dishes prepared. Timing and quantity of protein substitute consumed were also recorded. Nutritional analysis of food intake was calculated using the Microdiet computer programme based on *McCance & Widdowson 's "The Composition of Foods "* (Holland *et al,* 1991), with supplementary analysis data provided by the Royal Society of Chemistry (Holland *et al,* 1991a; Holland, Unwin, and Buss, 1991b; Holland Unwin, and Buss, 1989; Holland, Unwin, and Buss, 1998). Additional information on protein substitutes and low protein special products was provided by manufacturers and added to the database. Energy intake was calculated as a percentage of the estimated average requirement for energy (EAR) (DH, 1991) and nutrient intake as a percentage of reference nutrient intake (RNI).

### (b) Assessment of plasma phenylalanine concentrations

Carers took weekly skin puncture bloods specimens at home. In addition the previous four blood phenylalanine concentrations before study commencement were recorded. Blood specimens were taken at the same time of day, pre-breakfast, under fasting conditions. The blood specimens were collected in Starstedt CB300 heparinised tubes (300 µl) and immediately posted to the hospital. Blood specimens were analysed for phenylalanine by high-pressure liquid chromatography (HPLC) by the hospital-screening laboratory.

### (c) Assessment of biochemical and haematological status

Venous blood samples were collected at study entry and at the end of eight weeks for haemoglobin, iron, zinc, copper, calcium, zinc, copper, electrolytes and albumin.

### (d) Assessment of Anthropometry

Anthropometric data were collected at study entry and week 8. Weight to the nearest 10g, was measured using Seca electronic scales. Length was recorded to the nearest lmm by measuring supine length by an infantometer in children less than 2 years, and standing height in children over 2 years using a Harpenden Stadiometer. Length/height measurements were then converted into z scores, which denotes units of standard deviation from the median.

### (e) Assessment of acceptability

At the end of week 8, subjects or carers rated on a smiley face chart their impression of the PKU Gel^{TM}. Overall appearance, taste, smell, texture, volume, packaging and preparation were all assessed.

Plasma phenylalanine concentrations pre-study (week -3 to 0) and post treatment (week 1 to 8) were compared by paired t tests. Differences in weight, height, and other biochemical and haematological parameters were compared by paired *t* tests.

### Results

### (a) Dietary intake

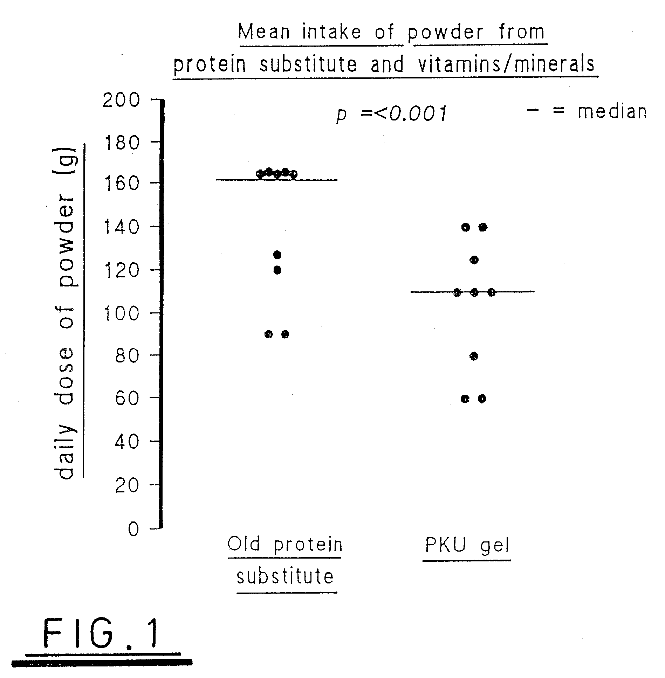

The median dose of PKU gel^{TM} daily was 110g daily (range 60g to 140g). Two subjects also took 5g Aminogran food Supplement daily. There previous median dose of protein substitute was higher at 160g daily (range 90g to 165g) and 3 took additional Aminogran food Supplement daily, as shown in Table 9 below and Figure 1 of the accompanying drawings.

**Table 9**

| Daily weight of protein substitute/vitamin and mineral powders pre and post PKU Gel^{TM} | | |
|---|---|---|
| **Initial of subject** | **Pre PKU-Gel** | **Post PKU-Gel** |
| JD | 165g | 110g |
| JF | 160g | 105g |
| DA | 165g | 125g |
| KL | 90g | 60g |
| RB | 164g | 140g |
| MC | 120g | 80g |
| TC | 164g | 140g |
| RW | 127 | 110g |
| CL | 90g | 60g |

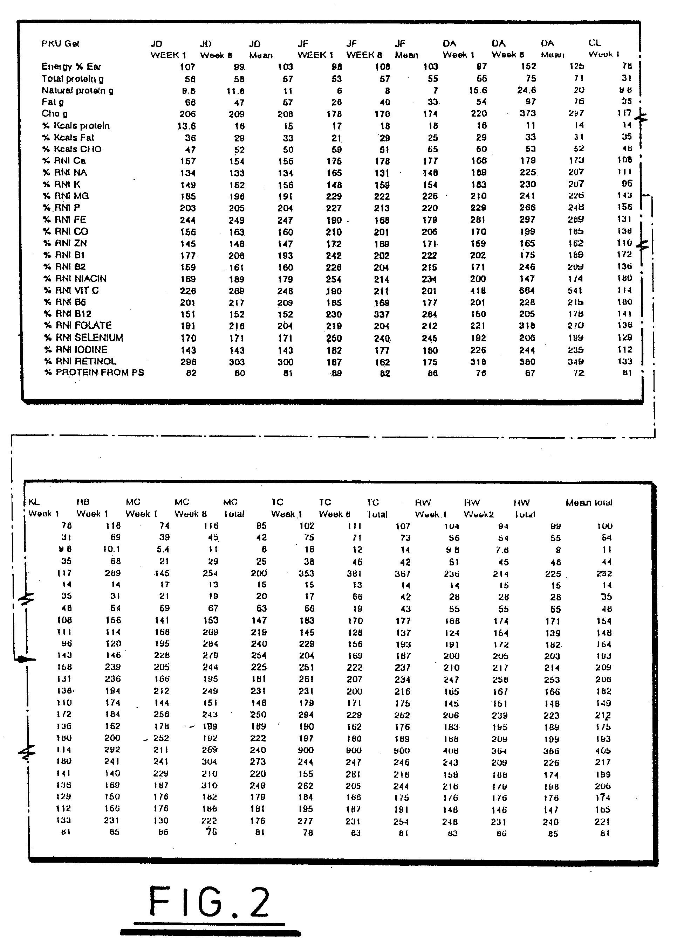

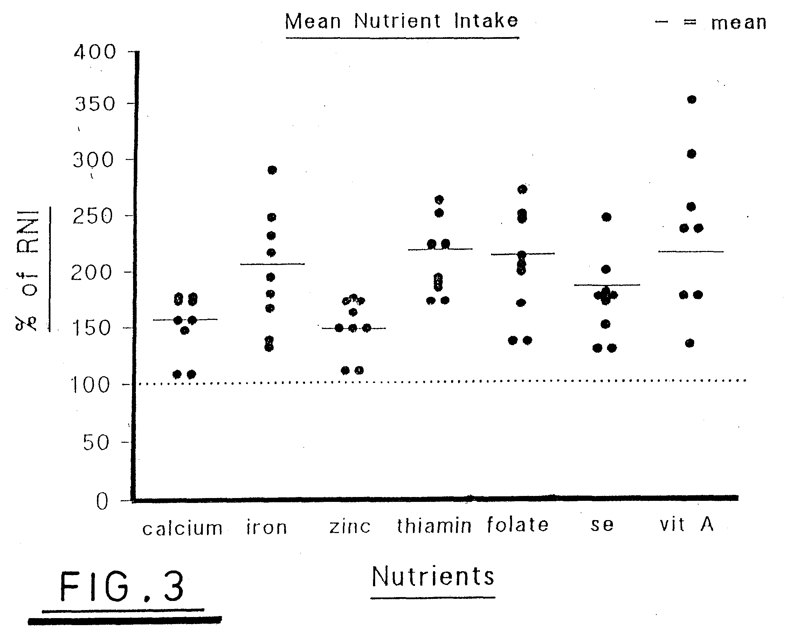

The intake of all nutrients, shown in Figures 2 and 3, exceeded DH (1991) dietary reference values but no nutrients appeared excessively high except one subject (TC) who had a particularly high vitamin C intake. He was from a vegan family and ate huge quantities of fruit and drank high intakes of fresh fruit juice. Seven of the children took the protein substitutes as a paste and two continued to take the protein substitute as a drink. The protein substitute provided a median of 81% (range 72% to 86%) of the total protein intake. It also provided a median of 376 kcals daily (range 205 to 479 kcal) daily.

### (b) Plasma Phenylalanine concentrations

The blood phenylalanine levels are shown in Table 10 below.

**Table 10**

| Blood Phenylalanine Levels | | | **umol/l** | | | | | | |
|---|---|---|---|---|---|---|---|---|---|
| Week | DA | RB | TC | MC | JD | JF | CL | KL | RW |
| Aim | 100-450 | 100-700 | 100-450 | 100-350 | 100-350 | 100-350 | 100-350 | 100-350 | 100-350 |
| -3 | 170 | 680 | 370 | 450 | 220 | 200 | 200 | 210 | 220 |
| -2 | 180 | 410 | 180 | 460 | 220 | 190 | 200 | 240 | 320 |
| -1 | 190 | 118 0 | 380 | 720 | 190 | 240 | 200 | 120 | 200 |
| 0 | 270 | 880 | 70 | 740 | 230 | 50 | 150 | 210 | 60 |
| 1 | 180 | 980 | 160 | 410 | 220 | 170 | 180 | 240 | 280 |
| 2 | 120 | 870 | 290 | 310 | 180 | 180 | 250 | 350 | 200 |
| 3 | 150 | 880 | 120 | 990 | 220 | 210 | 270 | 400 | 230 |
| 4 | 190 | 220 | 330 | 530 | 300 | 200 | 560 | 330 | 180 |
| 5 | 200 | 100 | 340 | 730 | 170 | 310 | 280 | 330 | 220 |
| 6 | 340 | 30 | 450 | 530 | 190 | 260 | 230 | 200 | 250 |
| 7 | 270 | 860 | 380 | 190 | 330 | 280 | 370 | 440 | |
| 8 | 300 | 810 | 390 | 130 | 220 | 200 | 330 | 340 | |
| 9 | 260 | 470 | 210 | 360 | 220 | 460 | 360 | 350 | |
| 10 | | | | 410 | | | | | |
| 11 | | | | 300 | | | | | |
| 12 | | | | 880 | | | | | |
| 13 | | | | 330 | | | | | |
| 14 | | | | 120 | | | | | |
| 15 | | | | 60 | | | | | |
| 16 | | | | 230 | | | | | |
| 17 | | | | 240 | | | | | |
| 18 | | | | 200 | | | | | |
| 19 | | | | 290 | | | | | |

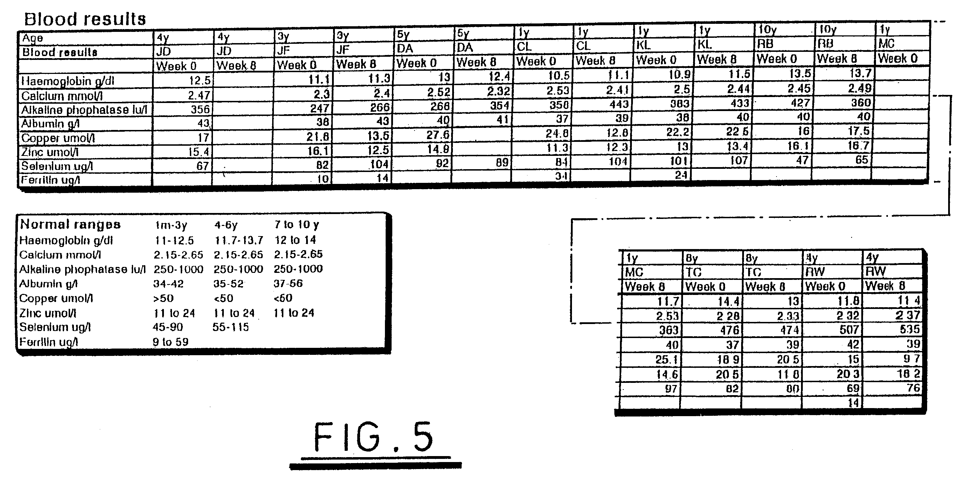

The mean plasma phenylalanine concentration in the 4 weeks prior to starting the study was 311µmol/l (SD 291) and during the 8 weeks of the study was 318 µmol/l (SD 122), as illustrated in Figure 5. There was no significant difference between the 2 groups. One child (RB) had slightly improved her blood phenylalanine concentrations whilst taking the PKU-Gel^{TM}, presumably because of better compliance. One child had worse control initially but this was associated with an intercurrent illness, which can adversely affect plasma phenylalanine concentrations due to catabolism. This child (MC) was studied for a longer follow-up period and his plasma phenylalanine concentrations returned to acceptable concentrations on the PKU-gel^{TM} when he recovered from his illness.

### (c) Biochemical and haematological status

No abnormal analytes were noted for any child at the start and end of trial (with the exception of copper in one subject (RW)), as shown in Figure 5. Although attempted, it was not possible to collect blood from one subject at the beginning of the trial and one subject refused to have a repeat blood sample at the end of the trial.

### (d) Anthropometry

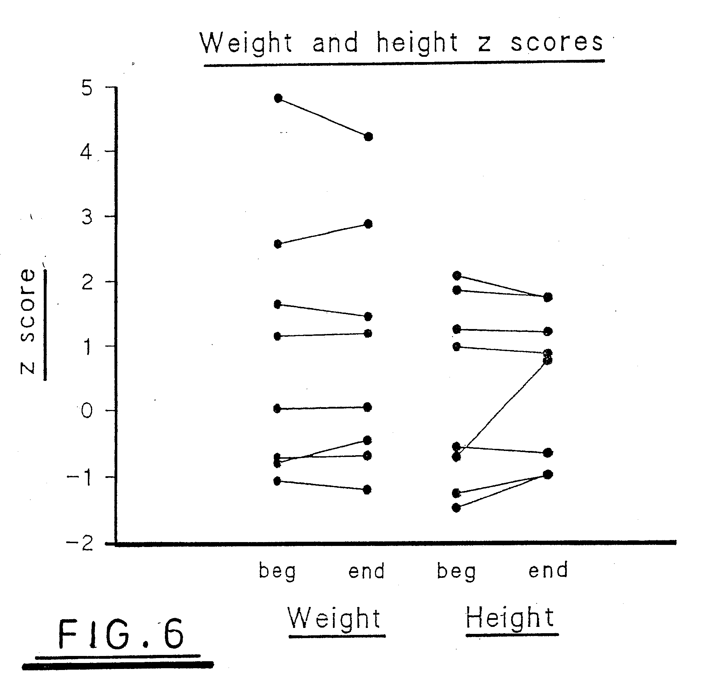

All children except one gained weight as shown in Table 11 below. The child who failed to gain weight would neither sit nor stand on the scales so the weight was probably inaccurate and not valid. All weight and heights (with the exception of the above child) was converted in to z scores (see Table 12 below and Figure 6 of the accompanying drawings). The mean z score for weight slightly increased between the study start and end from 0.92 (range -1.06 to 4.80) to 0.93 (-1.18 to 4.20). The mean z score for height also improved during the 8 week study period (mean week 0: 0.14 [range -1.45 to 2.08] to mean week 8: 0.36 [range -0.97 to 1.75].

**Table 11**

| | **Weight Week 1** | **Weight Week 8** | **Height Week 1** | **Height Week 8** |
|---|---|---|---|---|
| **DA** | 30.85 | 31.9 | 117.5 | 118.7 |
| **RB** | 26.7 | 26.5 | 134.7 | 134.7 |
| **TC** | 29.85 | 30.7 | 130.4 | 131.8 |
| **MC** | 11.6 | 12.17 | 85 | 85 |
| **JD** | 20.4 | 20.5 | 111.6 | 111.6 |
| **JF*** | 19.5 | 19.2 | 101.5 | 101* |
| **KL** | 10.22 | 10.7 | 76.8 | 79.4 |
| **CL** | 10.12 | 11 | 76.2 | 79.4 |
| **RW** | 25 | 26.4 | 112.5 | 112.9 |

| | | | | |
|---|---|---|---|---|
| * Difficult to weigh - had to be held. | | | | |

**Table 12**

| **Weight z score Study beginning** | **Weight z score Study end** | **Height z score Study beginning** | **Height z score Study end** |
|---|---|---|---|
| **4.8** | 4.2 | 1.86 | 1.75 |
| **-1.06** | -1.18 | -0.54 | -0.64 |
| **1.15** | 1.2 | -0.69 | 0.77 |
| **0.03** | 0.06 | 0.99 | 0.88 |
| **1.64** | 1.46 | 2.08 | 1.74 |
| **-0.79** | -0.44 | -1.45 | -0.97 |
| **-0.71** | -0.67 | -1.24 | -0.97 |
| **2.56** | 2.87 | 1.26 | 1.22 |

### (e) Tolerance and Acceptability

No subject complained of abdominal pain, nausea, diarrhoea, or vomiting whilst taking the PKU-gel^{TM}.

All nine subjects have requested to continue to use the PKU-gel^{TM} in preference to their previous protein subject. Seven subjects described the taste, texture and volume as 'very nice' or 'quite nice'. Only two of the children described the taste and smell as 'not so nice' or 'horrid' but they still took this protein substitute better than their previous protein substitute (see Table 13 below). All nine carers said the PKU-gel^{TM} was easy to prepare and eight carers/subjects said the volume was lower than previous protein substitutes. Five subjects preferred the raspberry PKU-gel^{TM}, two the unflavoured and one the orange flavour. Several advantages were listed for the PKU-gel^{TM}, given in Table 14 below, and these included convenience of being pre-measured in sachets, pleasant smelling, no need to give an extra vitamin and mineral supplement, less sticky and better consistency than other protein substitutes given as a paste. There were few disadvantages given although one carer commented they would prefer foil packaging and one child said it filled her up.

**Table 13**

| | **Very nice** | **Quite nice** | **O.K.** | **Not so nice** | **Horrid** |
|---|---|---|---|---|---|
| **Taste** | 56% | 22% | 11% | 0 | 11% |
| **Texture** | 56% | 22% | 11% | 11% | 0 |
| **Appearance** | 56% | 11% | 11% | 0 | 22% |

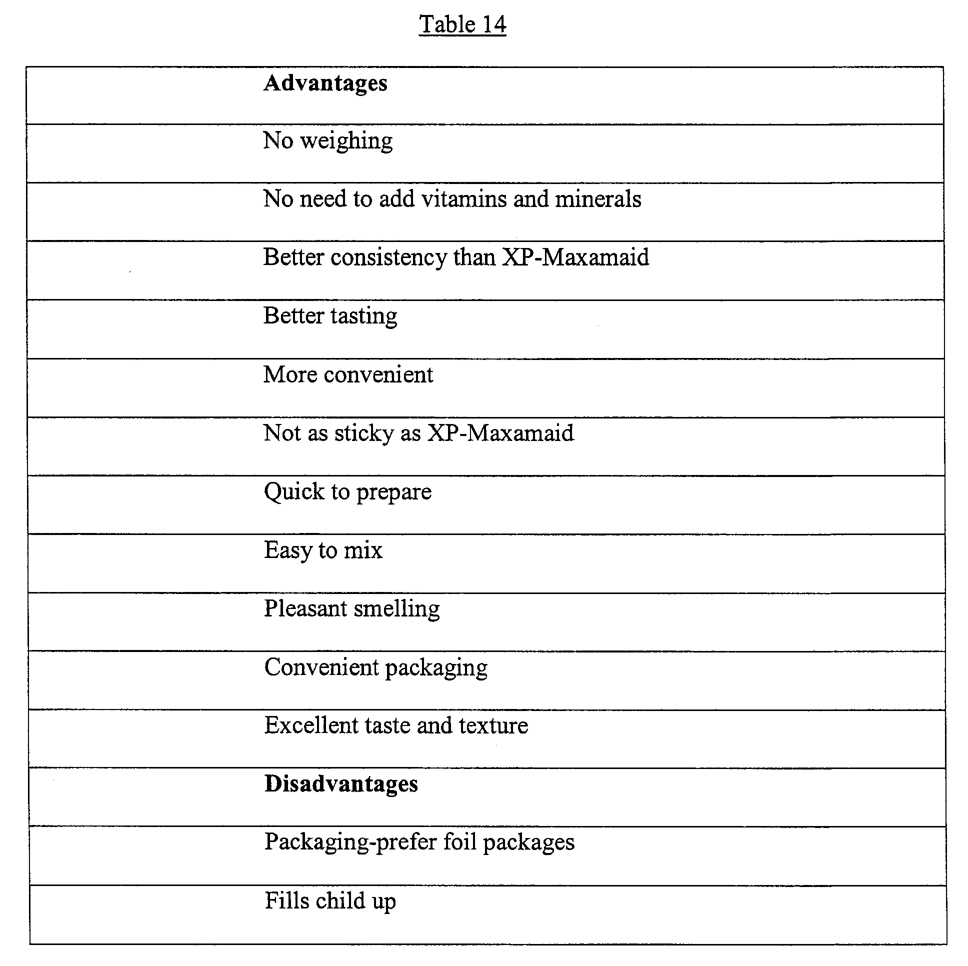

It is to be appreciated that alternative thickening agents could be included with the amino acid mixture in the protein substitute of the present invention, such as other modified starches, a gum or celluloses. The use of such agents with specific amino acid mixtures may be used to treat and/or manage patients suffering from other diseases which have their dietary protein substituted with a mixture of synthetic amino acids, such as other diseases of amino acid metalbolism (for example Maple Syrup Urine Disease (MSUD), Tyrosinaemia, Methylmalonia acid aemia, Homocystinurea, Glutaric aciduria, Isovaleric acidaemia and Hyperlysinaemia) or other diseases where amino acid mixtures are used in their treatment and/or management, such as cows milk protein allergy, short gut syndrome and gut resection patients.

The protein substitute of the present invention may be consumed in a number of ways depending upon the age and severity of the particular disease. For example, the substitute may be used as a weaning PKU protein substitute for infants, as a stand alone product or one which can be added to permitted foods. The gel-like substance may be used as a spread, for example being flavoured as a marmalade or jam or a chocolate (substitute) or savoury (e.g., cheese) spread that can be taken with low protein bread or crackers. Furthermore, the substitute may be flavoured and made up into a dessert, such as crème brulee, crème caramel or custard.

## Claims

1. An amino acid composition for administration to a patient for the treatment and/or management of a disease, the composition comprising an amino acid or specific combination of amino acids suitable for treatment of a particular disease and a thickening agent, such that addition of an aqueous liquid to the composition provides a smooth gel-like substance for consumption by the patient.

2. An amino acid composition as claimed in claim 1 wherein the thickening agent is included in amount of 1% to 40%.

3. An amino acid composition as claimed in claim 1 or claim 2 wherein the thickening agent includes pre-gelatinised starch.

4. An amino acid composition as claimed in claim 3 wherein the pre-gelatinised starch is included in an amount of at least 5%.

5. An amino acid composition as claimed in claim 1 or 2 wherein the thickening agent includes a modified starch.

6. An amino acid composition as claimed in claim 5 wherein the modified starch is selected from the group consisting of a starch ester, a starch phosphate, an oxidised starch, a mono starch phosphate, a di-starch phosphate, a phosphated di-starch phosphate, an acetylated di-starch phosphate, an acetylated starch or starch sodium octenyl succinate.

7. An amino acid composition as claimed in claim 5 or 6 wherein the modified starch is included in an amount of at least 5%.

8. An amino acid composition as claimed in claim 1, 2 or 3 wherein the thickening agent includes one or a combination of gums.

9. An amino acid composition as claimed in claim 8 wherein the gum is a locust bean gum, guar gum, Acacia gum, Xanthan gums and/or pectins.

10. An amino acid composition as claimed in claim 9 wherein the gum is included in an amount not less than 1%.

11. An amino acid composition as claimed in claim 1, 2 or 3 wherein the thickening agent includes one or a combination of celluloses.

12. An amino acid composition as claimed in any one of the preceding claims for use as a protein substitute.

13. An amino acid composition as claimed in claim 12 for the treatment or management of Phenylketonuria, Maple Syrup Urine Disease, Tyrosinaemia, Methylmalonic acidaemia, Homocystinurea, Glutaric aciduria, Isovaleric acidaemia, Hyperlysinaemia, Alcaptonuria, Urea Cycle Disorders, Propronic acidemia, Cows milk protein allergy, short gut syndrome or gut resection patients.

14. An amino acid composition as claimed in any one of the preceding claims wherein a natural or synthetic additive is included in the mixture to provide flavouring to the product.

15. An amino acid composition as claimed in any one of the preceding claims wherein water is added to the mixture to form the gel-like substance.

16. An amino acid composition as claimed in any one of the preceding claims further comprising minerals, trace elements and vitamins.

17. An amino acid composition as claimed in any one of the preceding claims wherein the mixture has a protein carbohydrate ratio of approximately 1:1.

18. A phenylalanine-free protein substitute comprising a specific combination of amino acids corresponding to the amino acid profile of normal dietary protein but excluding phenylalanine and a thickening agent such that addition of an aqueous liquid to the mixture provides a smooth gel-like substance for consumption by the patient.

19. A phenylalanine-free protein substitute as claimed is claim 18 wherein the thickening agent is selected from the group consisting of pre-gelatinised starch, modified starch, gums and/or celluloses.
